# EUROPEAN PATENT SPECIFICATION

(11) **EP 4 301 161 B1**
(45) Date of publication and mention of the grant of the patent: **29.01.2025**
(21) Application number: 22711342.0
(22) Date of filing: 23.02.2022
(51) Int. Cl.: A61K 31/201, A61K 31/202, A61K 31/23, A61K 47/12, A61K 47/14, A23L 33/115, A23L 33/12, A23L 33/17, A23L 33/185, A23L 33/21, A23L 33/00, A61K 31/19, A61K 31/733, A61P 3/02, A61P 25/08, A61P 25/28, A23L 33/15, A61K 9/00, A61K 36/48, A61P 25/06

(54) **LIQUID FOOD PRODUCT TO MANAGE THE KETOGENIC DIET**
FLÜSSIGES LEBENSMITTEL ZUR HANDHABUNG DER KETOGENEN DIÄT
PRODUIT ALIMENTAIRE LIQUIDE POUR LA GESTION DU RÉGIME CÉTOGÈNE

(30) Priority: 04.03.2021 IT 202100005009
(43) Date of publication of application: 10.01.2024
(73) Proprietor: Dr. Schär S.P.A., 39014 Postal (IT)
(72) Inventor: CERNE, Virna Lucia, 34011 DUINO-AURISINA (IT); BARBAN, Fabio, 33100 UDINE (IT)
(74) Representative: Petraz, Davide Luigi
(86) International application number: PCT/IT2022/050031
(87) International publication number: WO 2022/185349

(56) References cited:
- WO-A1-2014/008692
- US-A1- 2013 157 937
- US-A1- 2018 110 241
- LEI ENIE ET AL: "Fatty acids and their therapeutic potential in neurological disorders", NEUROCHEMISTRY INTERNATIONAL, ELSEVIER, AMSTERDAM, NL, vol. 95, 3 March 2016 (2016-03-03), pages 75 - 84, XP029511128, ISSN: 0197-0186, DOI: 10.1016/J.NEUINT.2016.02.014
- JANA ORSAVOVA ET AL: "Fatty Acids Composition of Vegetable Oils and Its Contribution to Dietary Energy Intake and Dependence of Cardiovascular Mortality on Dietary Intake of Fatty Acids", INTERNATIONAL JOURNAL OF MOLECULAR SCIENCES, vol. 16, no. 6, 5 June 2015 (2015-06-05), pages 12871 - 12890, XP055380982, DOI: 10.3390/ijms160612871
- AVGERINOS KONSTANTINOS I ET AL: "Medium Chain Triglycerides induce mild ketosis and may improve cognition in Alzheimer's disease. A systematic review and meta-analysis of human studies", AGEING RESEARCH REVIEWS, ELSEVIER, AMSTERDAM, NL, vol. 58, 20 December 2019 (2019-12-20), XP086064438, ISSN: 1568-1637, [retrieved on 20191220], DOI: 10.1016/J.ARR.2019.101001
- MANCINI ANNAMARIA ET AL: "Biological and Nutritional Properties of Palm Oil and Palmitic Acid: Effects on Health", MOLECULES, vol. 20, no. 9, 18 September 2015 (2015-09-18), pages 17339 - 17361, XP055840488, DOI: 10.3390/molecules200917339

## Description

### FIELD OF THE INVENTION

Embodiments described here concern a liquid food product, that is, a beverage, intended preferably but not only for individuals suffering from particular pathological conditions, in particular neurological conditions, on the basis of which a diet low in carbohydrates and rich in fats can be recommended. In particular, the liquid food product according to the present description is suitable to manage the ketogenic diet.

### BACKGROUND OF THE INVENTION

It is known that the pathophysiological processes that trigger and characterize particular morbid conditions, whether they are diseases, syndromes or other, can involve and in particular can alter the pathways that regulate the metabolism of at least one type of cells that make up the organ or apparatus in which the morbid condition develops.

Since the metabolism of the cells is at least partly a function of the foods eaten, it is possible, through an adequate diet, to reduce and correct the symptoms of the disease or syndrome.

For some particular pathological conditions, particular dietary regimes can therefore be recommended and advantageous, able to reduce morbid symptoms.

These diets include the so-called "ketogenic diet", a term with which we typically refer to a category of diets intended to promote the formation of so-called "ketone bodies" in the organism by stimulating the process of ketogenesis.

The term ketone bodies typically refers to three acid compounds present in the blood in small quantities, that is, acetone (propanone), acetacetic acid (3-oxobutanoic acid) and beta-hydroxybutyric acid (3-hydroxybutanoic), which in particular conditions can be metabolized by the cells of the organism, as an alternative to carbohydrates, to provide the necessary energy supply.

These compounds derive from the metabolism of fats introduced with food and are of fundamental importance, in particular, for the correct function of brain cells, since they are the only compounds, in addition to glucose, that can be used by these cells to produce energy.

Therefore, in the event that an organism has insufficient glucose levels, a condition called ketosis is established, that is, an increase in ketone bodies in the blood, such that the brain cells still receive nourishment.

Since the condition of ketosis can be "artificially" induced by following a ketogenic diet, that is, a diet low in carbohydrates and high in fats, this diet has been applied and studied to manage and/or correct the metabolism of brain cells, in order to treat individuals with neurological diseases.

In particular, clinical studies have highlighted how the condition of ketosis that is established by regularly following the ketogenic diet can be applied to manage neurological diseases including epilepsy or neurodegenerative diseases that affect the central nervous system (CNS).

The first evidence of the efficacy of ketosis in reducing the symptoms of neurological diseases was highlighted by the positive and encouraging results obtained from the ketogenic diet in the treatment of drug-resistant epilepsy ("A multicenter study of the efficacy of the ketogenic diet", Vining EP, Freeman JM, Ballaban-Gil K, Camfield CS, Camfield PR, Holmes GL, Shinnar S, Shuman R, Trevathan E, Wheless JW, in Arch. Neurol., 1998 Nov; 55(11): 1433-7).

Epilepsy is a neurological disease of the CNS, for which there can be many causes, in which the activity of the nerve cells in the brain is interrupted, causing the individual to have seizures, unusual behavior and loss of consciousness, and which affects individuals of any age, with a higher incidence in infancy and the elderly.

In cases of chronic epilepsy, it is usually treated with anti-epileptic drugs, but about 30% of patients develop the drug-resistant form of the disease.

For these patients and for those who cannot take drugs also due to their side effects, such as for example specific allergies to the active ingredient or to the excipients, the control of the symptoms of the disease can be managed by following a ketogenic diet.

It also appears that the ketogenic diet may be useful in managing and/or assisting the treatment of other diseases of the CNS, such as Alzheimer's disease, Parkinson's disease, autism, multiple sclerosis, migraines, trauma and brain neoplasms.

Although the mechanisms of action remain at least partly unknown, some experimental evidence shows that its efficacy is closely correlated to the normalization of the conditions of energy metabolism altered by these pathological conditions ("Pathophysiology of Epilepsy in Autism Spectrum Disorders", Stafstrom CE, Hagerman PJ, Pessah IN, Editors; "Jasper's Basic Mechanisms of the Epilepsies" [Internet], 4th edition, Bethesda (MD): National Center for Biotechnology Information (US), 2012.).

It has also been shown that the increase in the concentration of ketone bodies in the blood produces a neuro-protective effect, since it increases the level of ATP, reducing the production of ROS (Reactive Oxygen Species), through the intensification of the oxidation of NaOH and inhibition of mitochondrial membrane transition ("Ketone bodies are protective against oxidative stress in neocortical neurons", Kim DY, Davis LM, Sullivan PG, Maalouf M, Simeone TA, van Brederode J, Rho JM; J Neurochem., 2007 June; 101(5): 1316-26. Epub 2007 Mar 30).

*In vitro* studies have also demonstrated a protective effect carried out by the ketone bodies against these mitochondrial dysfunctions ("Ketones prevent synaptic dysfunction induced by mitochondrial respiratory complex inhibitors", Kim DY, Vallejo J, Rho JM, J Neurochem; 2010 Jul; 114(1): 130-41).

In the ketogenic diet, in order to promote ketogenesis, the nutrients taken must be carefully balanced according to the so-called ketogenic ratio, that is, in such a way that the quantity of lipids supplied to the organism is prevalent compared to the sum of proteins and carbohydrates, for example substantially double, or even more, for example even multiples, compared with the sum of proteins and carbohydrates.

To promote this aspect, there are food products on the market that have formulations with a nutrient ratio compatible with the purposes of the ketogenic diet.

One disadvantage of known food products is that the need to guarantee the ketogenic ratio can be detrimental to other nutritional or organoleptic properties of the products themselves, which therefore can be difficult to accept, for example by children.

In particular, one possible disadvantage of known food products is that the need to guarantee the ketogenic ratio, that is, the strong prevalence of fats, in particular long-chain triglycerides, may require the organism to make a considerable effort in order to metabolize them.

Another disadvantage is that known food products cannot be consumed by certain individuals, due to the presence of comorbidities.

Yet another disadvantage of current formulations is the presence of allergens, such as for example milk or soy proteins, which therefore make these products less usable by patients with food intolerances or allergies.

It is also known that the food products generally used for the ketogenic diet are low in water and are associated with a diet already rich in poorly hydrated foods.

A further disadvantage is, therefore, that adverse events occur due to poor hydration, especially in the more susceptible age groups, for example infants and the elderly, and/or in cases where the patient does not independently take liquids in another form.

Document US-A-2018/110241 describes an aqueous ketogenic composition, for example to manage drug-resistant epilepsy, which includes water, hydrolyzed proteins, medium-chain triglycerides (MCTs), seed or nut oil, emulsifier, chelating agent, sweetener, carbohydrates, antioxidants, vitamins and minerals. This composition is fiber free and has a ketogenic ratio, that is, the ratio by weight of fats to the combined weight of proteins and carbohydrates, from 3:1 to 4:1.

Document US-A-2013/157937 describes a high-fat liquid formulation for a ketogenic diet, which comprises casein and food fibers, is MCT-free and has a ketogenic ratio of 3.5:1.

Document WO-A-2014/008692 describes a milk formula for a ketogenic diet which contains, per 100g of product: 1.5-12.5g of fats, 0-5g of protein, 0-0.5g of carbohydrates, 0-0.6g of aromas, 0-1.2g of nourishing essences and the remainder is liquid milk.

There is therefore a need to perfect a food product that can overcome at least one of the disadvantages of the state of the art.

In particular, one purpose of the present invention is to provide a food product to manage the ketogenic diet and in particular effective in treating the symptoms of neurological diseases.

Another purpose is to provide a food product that has a formulation that reduces the side effects associated with the ketogenic diet and at the same time is at least as effective as known products.

Another purpose of the present invention is to provide a food product that can be consumed and assimilated by individuals with possible comorbidities and/or other pathological conditions other than those which have made the ketogenic diet necessary.

Another purpose of the present invention is to provide a food product in which the ketogenic ratio between lipids, proteins and carbohydrates is guaranteed, and the nutritional and organoleptic properties are adequate and satisfied.

Furthermore, another purpose of the present invention is to provide food products which are more balanced, from a nutritional point of view, than the known products used to manage the ketogenic diet.

Another purpose of the present invention is to provide food products which are more digestible than the products known in this specific field.

Another purpose of the present invention is also to provide a liquid food product for the ketogenic diet which is easy and immediate to consume.

Another purpose is to provide a food product to manage the ketogenic diet which can also be used by patients with food intolerances or allergies.

Another purpose of the present invention is to provide a food product to manage the ketogenic diet which helps to counteract the problems deriving from a possible condition of dehydration.

Another purpose of the present invention is to provide a food product to manage the ketogenic diet which does not require the body to make a considerable effort to digest and assimilate lipids.

Yet another purpose is to provide a food product for use in the treatment of neurological diseases, such as, for example, epilepsy, neurodegenerative diseases such as Alzheimer's disease and Parkinson's disease, or autism, multiple sclerosis, migraines, trauma and brain neoplasms.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the present invention or variants to the main inventive idea.

In accordance with the above purposes, the embodiments described here concern a liquid food product, that is, a beverage, suitable to manage the ketogenic diet, which overcomes the limits of the state of the art and eliminates the defects present therein.

In the embodiments described here, the liquid food product comprises:
- lipids between 10% and 20% by weight with respect to the total weight of the product, of which short-chain fatty acids (SCFAs) or their salts or their esters, between 0% and 1% by weight with respect to the total weight of the product, and medium-chain triglycerides (MCTs), between 10% and 16% by weight with respect to the total weight of the product;
- carbohydrates less than 2% by weight with respect to the total weight of the product;
- fibers between 1% and 6% by weight with respect to the total weight of the product;
- proteins between 5% and 10% by weight with respect to the total weight of the product;
- water between 60% and 75% by weight with respect to the total weight of the product.

The presence of MCTs in the product described here allows to obtain advantages with regards to the organoleptic aspect, maintaining a low ketogenic ratio and at the same time improving the sensory aspect of the product, in favor of a greater adherence to the ketogenic diet by those who take the product described here, as described in detail below.

Furthermore, the presence of fibers, in particular prebiotic fibers, in the formulation described here is advantageous since they are the ideal substrate for the formation of endogenous SCFA by the intestinal microbiota. This aspect, together with the presence of MCTs, allows to obtain significant nutritional aspects, as explained in detail below.

Furthermore, in such embodiments, the liquid food product can have a ketogenic ratio between lipids and the sum of carbohydrates and proteins comprised between 1.5:1 to 2.5:1.

In such embodiments, the lipids can comprise unsaturated fatty acids, or their salts, or their esters, in particular polyunsaturated fatty acids (PUFAs) or their salts, or their esters.

The unsaturated fatty acids can also be supplied in the form of unsaturated fatty acid salts, unsaturated fatty acid triglycerides, or other unsaturated fatty acid esters.

In some embodiments, the liquid food product comprises, as a source of lipids, in particular of unsaturated fatty acids, one or more substances selected from the group comprising: safflower oil, linseed oil, animal or vegetable oil rich in docosahexaenoic/docosahexaenoate acid (DHA), or combinations thereof.

Advantageously, the liquid food product described here, in particular thanks to the presence of MCTs and possibly SCFAs, is suitable to manage the ketogenic diet and it is also, at the same time, nutritionally more balanced, more digestible and with a better composition of nutrients and ingredients compared to other solutions known in the state of the art.

The Applicant has found, in particular, that the presence of MCTs allows to obtain a double effect, since it advantageously allows both to keep the ketogenic ratio low, between 1.5:1 and 2.5:1 (for example 2:1), and also to improve the sensory aspect of the product, promoting greater adherence to the diet. Ketogenic diets with a higher ratio, for example from 3:1 to 4:1, are more difficult to follow (see scientific publication "Efficacy of and Patient Compliance with a Ketogenic Diet in Adults with Intractable Epilepsy: A Meta-Analysis"; Journal of Clinical Neurology 2015; 11(1): 26-31).

In some embodiments, the medium-chain triglycerides (MCTs) as above comprise medium-chain triglycerides (MCTs) deriving from decanoic acid (C10), octanoic acid (C8) and lauric acid.

Favorably, the formulation described here can provide a C8-C10 ratio, that is, a ratio between MCTs deriving from octanoic acid (C8) and MCTs deriving from decanoic acid (C10), between 60:40 and 40:60, which further helps the sensory improvement described above in the formulation described here.

Furthermore, the Applicant has found that the presence of MCTs, in particular of C10, can increase paracellular transport at the level of the intestinal epithelium. In fact, it relaxes cell-cell contacts, acting on tight junction (TJ) proteins, proteins designed to bond the cells of the membrane epithelium, making it almost impermeable to the uncontrolled passage of substances. At the same time, the presence of prebiotic soluble fiber is favorably the ideal substrate for the formation of endogenous SCFA by the intestinal microbiota. Advantageously, the presence of MCTs in the ratios provided, and the fact that the production of endogenous SCFA by the intestinal microbiota is promoted due to the presence of fibers in the formulation, regardless of the possible addition of exogenous SCFA, can have an important synergistic effect in the reduction of side effects and in improving the effects of the ketogenic diet. In fact, the SCFAs activate signaling cascades, acting as ligands of G protein-coupled receptors that can modulate the inflammatory response and increase the integrity of the intestinal barrier. These tight junctions, located in the most apical zone of the epithelial cells, control the diffusion of ions, macromolecules and the entry of microorganisms from the intestinal lumen into the tissues (see scientific publication "Use of Short-Chain Fatty Acids for the Recovery of the Intestinal Epithelial Barrier Affected by Bacterial Toxins", Diliana Pérez-Reytor et.al. 2021).

### BRIEF DESCRIPTION OF THE DRAWINGS

These and other aspects, characteristics and advantages of the present invention will become apparent from the following description of some embodiments, given as a non-restrictive example with reference to the attached drawings wherein:
- figs. 1a and 1b show two tables which describe example compositions (Table 1a) and energy and nutritional characteristics (Table 1b) which relate to an example embodiment of the liquid food product according to the present description;
- figs. 2a and 2b show two tables which describe example compositions (Table 2a) and energy and nutritional characteristics (Table 2b) which relate to an example embodiment of the liquid food product according to the present description.

It is understood that elements and characteristics of one embodiment can conveniently be incorporated into other embodiments without further clarifications.

### DESCRIPTION OF SOME EMBODIMENTS

We will now refer in detail to the possible embodiments of the invention, of which one or more examples are shown in the attached drawings, by way of a non-limiting illustration. The phraseology and terminology used here is also for the purposes of providing non-limiting examples.

Unless otherwise defined, all the technical and scientific terms used here and hereafter have the same meaning as commonly understood by a person with ordinary experience in the field of the art to which the present invention belongs.

Even if methods and materials similar or equivalent to those described here can be used in practice and in the trials of the present invention, the methods and materials are described hereafter as an example. In the event of conflict, the present application shall prevail, including its definitions. The materials and examples have a purely illustrative purpose and shall not be understood restrictively.

Furthermore, all percentages, fractions and ratios between the different components are to be understood as fractions by weight (w/w) with respect to the total composition, even if not explicitly indicated, unless otherwise indicated. All the percentage intervals reported here are provided with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

In general, whenever reference is made to chemical species that can be ionized in an aqueous environment, such as for example carboxylic acids, it is understood that the name chosen to indicate the species also includes the corresponding ion or the corresponding salt, unless otherwise specified.

In this sense, it is not essential for the creation of the invention to specify the counter-ion of the salt, since it is obvious that, in those cases relevant to the present description, the aqueous solutions always include species suitable to act as counter-ion. For example, the use of the term "carboxylic acid or its salt" implies that the carboxylic acid can also be present as a carboxylate.

To the extent that the publications referenced here give a definition for a term that is in conflict with the definition implicitly or explicitly used in the present description, the definition used in the present description shall prevail.

Some embodiments of the invention described here concern a liquid food product, that is, a beverage, suitable to manage a ketogenic diet.

According to some embodiments, the invention is aimed, in particular but not exclusively, at individuals who need to follow a ketogenic diet.

Even more in particular, the invention is suitable to treat individuals suffering from morbid neurological conditions for which a ketogenic diet is recommended in support of pharmacological therapy and/or surgical therapy, or as a replacement for at least one of such therapies.

In accordance with some embodiments, the liquid food product is water-based, therefore ready for immediate use, and with a nutrient ratio compatible with, and functional for, the rules of the ketogenic diet.

The liquid food product according to the present description is advantageous in that it does not require further transformations by the consumer, and it is easy and immediate to take.

The embodiments of the invention provide an appropriate combination of substances to manage, effectively and with better results compared to known food products, symptoms connected to neurological diseases, such as, for example, epilepsy, neurodegenerative diseases such as Alzheimer's disease and Parkinson's disease, or autism, multiple sclerosis, migraines, trauma and brain tumors.

In some embodiments, the liquid food product has a water content comprised between 60% and 75% by weight with respect to the total weight of the product.

This characteristic can be particularly advantageous for certain classes of consumers suffering from particular neurological diseases such as, for example, subjects suffering from epilepsy, who may suffer from dehydration.

To the advantage of this, a formulation with pleasant organoleptic characteristics such as those provided by the invention can facilitate this function.

In some embodiments, the liquid food product comprises a fraction of lipids between 10% and 20% by weight with respect to the total weight of the product.

The term "lipids" and its derivatives, such as for example "lipid fraction", is understood here in the sense commonly used in the field of the art, with particular reference to the IUPAC (International Union of Pure and Applied Chemistry) definition of "substances of biological origin soluble in non-polar solvents" (source: PAC, 1995, 67, 1307, "Glossary of class names of organic compounds and reactivity intermediates based on structure", IUPAC Recommendations 1995, page 1348).

In some embodiments, the lipids comprise fats, in particular glycerides, that is, esters of glycerol with fatty acids.

Here, as fatty acids we include carboxylic acids with aliphatic chains, both saturated and unsaturated, as well as branched and unbranched, comprised between 4 and 28 atoms of carbon, although, similarly to what is reported in PAC, 1995, 67, 1307, "Glossary of class names of organic compounds and reactivity intermediates based on structure", IUPAC Recommendations 1995, page 1335, sometimes, by extension, they can comprise any aliphatic carboxylic acid.

What stated above for chemical species that can be ionizable in an aqueous environment is applied to fatty acids.

Therefore, here and in the present description, the term "lipids" and its derivatives, such as "lipid fraction", is used in a broad sense to include not only esters, in particular mono-, di- or triglycerides, of fatty acids, but also fatty acids, for example short- or medium-chain, or their salts.

The lipids, in particular the glycerides, can comprise simple monoglycerides, and/or diglycerides and/or triglycerides, that is, comprising a single type of fatty acid, or mixed, that is, comprising fatty acids of different types.

It is therefore understood that the phrase "fatty acid or its ester" is used with particular reference to the fact that the fatty acid can be esterified with glycerol, and therefore comprises at least all the esters obtainable by combining the fatty acid with glycerol.

Furthermore, here and in the present description, the free fatty acids, not necessarily esterified, and the corresponding salts are also considered as forming part of the lipid fraction, since they can potentially be combined with glycerol to form mono-, di- or triglycerides.

This type of molecules is also sometimes classified as a class of fatty acyl lipids, as for example reported for example in "Lipid Concentrations in Standard Reference Material (SRM) 1950: Results from an Interlaboratory Comparison Exercise for Lipidomics", NIST (National Institute of Standards and Technology), and in the publication "A comprehensive classification system for lipids", Eoin Fahy et al., Journal of Lipid Research 46(5): 839-61, June 2005.

In some embodiments, the lipids comprise medium-chain fatty acids (MCFAs), or their salts or their esters, in particular in the form of triglycerides of medium-chain fatty acids (MCFAs), also known as medium-chain triglycerides (MCTs), between 10% and 16% by weight with respect to the total weight of the product.

In some embodiments, at least 70% by weight of the lipid fraction of the liquid food product consists of medium-chain triglycerides (MCTs).

One advantage related to the use of MCTs is that they can be metabolized more rapidly compared to long-chain triglycerides (LCTs), typically used in other known solutions.

In particular, MCTs, unlike LCTs, can be metabolized even without the intervention of lipase and also reach the liver directly through the portal venous system, without passing through the lymphatic system ("Medium-chain Triglycerides in Pediatric Practice", Gracey et al., 1970 Aug; 45(242): 445-452.). Taking MCTs therefore increases the concentration of ketone bodies in the blood more rapidly.

Furthermore, the MCTs positively affect the intestinal microbiota ("Gut Microbiota and Metabolic Health: The Potential Beneficial Effects of a Medium-chain Triglyceride Diet in Obese Individuals", Ahmed Rial et al., Review 2016 May 12; 8(5): 281).

Advantageously, moreover, the MCTs have antimicrobial properties, for example they inhibit the proliferation of some fungal species ("Medium-chain triglycerides inhibit growth of Malassezia: implications for prevention of systemic infection", Papavassilis C., Mach KK., Mayser PA., Crit. Care Med., 1999 Sep, 27(9): 1781-6) and some fecal bacteria ("Protective effects of medium-chain triglycerides on the liver and gut in rats administered endotoxin", Kono H., Fujii H., Asakawa M., Yamamoto M., Matsuda M., Maki A., Matsumoto Y., Ann. Surg., 2003 Feb, 237(2): 246-55), they can be effectively employed in the treatment of LPS (lipopolysaccharide) mediated endotoxemia and can play a role in the reduction of intestinal inflammatory states ("Fatty acids, inflammation and intestinal health in pigs", Liu Y., J. Anim. Sci. Biotechnol., 2015 Sep, 9; 6(1): 41, doi: 10.1186/s40104-015-0040-1. eCollection 2015).

In some embodiments, which can be combined with all the embodiments described here, the medium-chain fatty acids (MCFAs) that make up the MCTs can comprise caproic acid (hexanoic acid), caprylic acid (octanoic acid), capric acid (decanoic acid), lauric acid (dodecanoic acid), or their combinations, or their salts, or their esters.

In some embodiments, the medium-chain triglycerides (MCTs) can comprise medium-chain triglycerides (MCTs) derived from decanoic acid (C10), octanoic acid (C8) and lauric acid.

In some embodiments, the product as above provides a ratio between MCTs deriving from octanoic acid (C8) and MCTs deriving from decanoic acid (C10) between 60:40 and 40:60. An advantageous example is 50:50. The Applicant has found that a ratio from 40:60 to 60:40 surprisingly helps the sensory improvement in the formulation described here in combination with the presence of MCTs which allow to maintain a low ketogenic ratio between 1.5:1 and 2.5:1 (for example 2:1) as described above.

Furthermore, decanoic acid, advantageously present in suitable quantities and proportions, proves to be particularly effective in the management of neurological pathologies, in particular epilepsy ("Seizure control by decanoic acid through direct AMPA receptor inhibition", Chang et al., Brain 2016 Feb, 139(Pt 2): 431-43).

Furthermore, a balanced ratio between octanoic acid and decanoic acid, keeping their quantities at levels proven to be effective for the management of neurological diseases, allows to reduce the side effects on the digestive system.

In particular, decanoic acid can increase paracellular transport at the level of the intestinal epithelium. In fact, it relaxes cell-cell contacts, acting on tight junction (TJ) proteins, proteins designed to bond the cells of the membrane epithelium, making it almost impermeable to the uncontrolled passage of substances.

The action of decanoic acid allows to expand these proteins, allowing the passage of nutrients between the cells, for example ionized or high molecular weight compounds, which would otherwise not pass. In fact, following absorption, C10 activates phospholipase C, a cytoplasmic phosphodiesterase that cleaves phosphatidyl-inositol 4,5-diphosphate (membrane phospholipid present on the inner sheet) generating two second messengers: diacylglycerol (DAG) and inositol-triphosphate (IP3). The latter messenger acts on calcium deposits (Ca2+) stored at the endoplasmic reticulum level, promoting the efflux of the ion into the intracellular compartment. The high concentration of calcium in the cytoplasm, which is normally absent, acts as an activator for a regulatory protein called calmodulin. The Ca2+/calmodulin complex binds to a series of enzymes, resulting in their activation. Among these, MLCK (Myosin light-chain kinase) is also induced, which phosphorylates myosin, thus allowing its interaction with actin. The result is the contraction of the TJs, their opening and therefore paracellular transport ("Physiological mechanism for enhancement of paracellular drug transport", Hayashi et al., 1999, 141.148).

In some embodiments, the lipids comprise short-chain fatty acids (SCFAs), or their salts or their esters, in particular in the form of triglycerides of short-chain fatty acids (SCFAs), also called short-chain triglycerides (SCTs), between 0.1% and 1% by weight with respect to the total weight of the product.

As stated, in some embodiments the SCFAs can be supplied in the form of esters, more in particular glycerol esters (triglycerides).

In the embodiments in which the short-chain fatty acids (SCFAs) are supplied as esters, only one or more but not all, or all, of the substituent groups of ester are SCFAs.

In particular, in the case of glycerol ester, only one or more but not all, or all, of the substituent groups that are bound to the glycerol to produce the ester are SCFAs.

In a preferential embodiment, it is advantageous that not all the substituent groups, which are bound to the alcohol to give the ester, are SCFAs. The Applicant has found that this preferential embodiment allows to solve the unpleasant taste that an ester in which all the substituent groups are SCFAs could have. For example, in the case of glycerol, only one, or at most two, of the substituent groups that are bound to the glycerol molecule to produce the ester are SCFAs. The remaining substituent groups can be medium-chain fatty acids (MCFAs) or long-chain fatty acids (LCFAs). For example, one substituent group are SCFAs and the second and third substituent groups are a medium-chain fatty acid (MCFA) or a long-chain fatty acid (LCFA). In another example, two substituent groups are SCFAs, and the third substituent group is a medium-chain fatty acid (MCFA) or a long-chain fatty acid (LCFA).

In some embodiments, the short-chain fatty acids (SCFAs), or their salts or their esters, can comprise butyric acid (butanoic acid), or its salt or its ester, for example supplied as sodium butyrate.

In such embodiments, the lower extreme value of the sodium butyrate range is sufficient to obtain the desired effects.

Butyric acid can, advantageously, keeping its dosages low, represent a powerful tool with which to improve the positive effects of the ketogenic diet and at the same time reduce its negative effects.

In fact, based on a mechanism of action conceptually opposite to that described above for decanoic acid, butyric acid can perform the function of counteracting the expansion of the TJs, proving to be very important for managing the microbiota, regulating dysbiosis and maintaining membrane integrity. In particular, butyric acid reduces the possibility that potentially harmful substances, usually destined to remain in the intestinal lumen, go outside of it and cause damage to the body and the intestine itself.

Specifically, an *in vitro* analysis ("Sodium Butyrate Promotes Reassembly of Tight Junctions in Caco-2 Monolayers Involving Inhibition of MLCK/MLC2 Pathway and Phosphorylation of PKC2", Miao et al., 2016 Oct 10; 17(10): 1696) highlights that the ability of butyrate to reassemble TJs derives primarily from the activation of a protein kinase (Calcium/calmodulin-dependent protein kinase, CaMKKβ), which in turn phosphorylates the AMPK effector (adenosine monophosphate - activated protein kinase). The latter acts by blocking MLCK and thus preventing the contraction mechanism at the level of the TJs.

The exogenous intake of butyric acid, provided by the liquid food product described here, can compensate for a possible reduction in the endogenous production of SCFAs due to the altered bacterial flora, directly supporting the increase in the level of ketosis.

In accordance with some embodiments, therefore, the presence of SCFAs (both endogenous SCFA produced by the intestinal microbiota due to the presence of fibers, and also possibly added in exogenous form in the formulation described here) and MCTs, in the ratios provided by the present invention, can have an important synergistic effect in reducing side effects and improving the effects of the ketogenic diet.

In particular, the combination of decanoic acid and sodium butyrate is advantageous for regulating the role of the former in increasing intestinal permeability, preventing the induction of paracellular transport mediated by them. On the other hand, the beneficial properties described for MCTs and SCFAs on the bacterial flora are preserved, amplifying their effect thanks to the synergistic use of the two.

From a quantitative point of view, the formulation provides a content of MCTs of at least 10%, the minimum limit required for the ketogenic use provided. For the SCFAs, it is reported in the literature that their functionality on TJs occurs at a much lower dosage. If they are present in the product described here, therefore, the range of use may be comprised between 0.1-1%.

In any case, the advantages described above given by the combination of MCTs and SCFAs are obtained even if the formulation described here does not include SCFAs, since in any case it comprises prebiotic fibers, which are the ideal substrate for the formation of endogenous SCFA by the intestinal microbiota, regardless of their addition, for example in the form of butyrate esters, salts or acids.

In some embodiments, the liquid food product comprises a carbohydrate fraction of less than 2%.

In this description, we refer to carbohydrates in the sense of carbohydrates that are digested by the body. Therefore, the definition of carbohydrates does not include those substances that, although they may have a chemical structure which can be associated with carbohydrates, are not metabolized, such as for example resistant starches, fibers and cellulosic substances.

In some embodiments, the carbohydrates comprise sugars less than 1% by weight.

In some embodiments, the carbohydrates, in particular sugars, can comprise sucralose and/or sweetener.

In some embodiments, which can be combined with all the embodiments described here, the carbohydrates can partly or totally come from other substances or their impurities.

In some embodiments, the sources of carbohydrates and/or sugars can comprise cocoa powder and/or flavorings to improve the organoleptic qualities.

Advantageously, this reduced content of carbohydrates, and in particular of sugars, allows to reach high ketogenic ratios, making the ketogenesis of the present food product more effective compared to similar known solutions.

In some embodiments, the liquid food product can be supplemented with non-digestible fibers and/or polysaccharides in order to stimulate the bacterial flora to produce endogenous SCFA.

In some embodiments, the food product can comprise fibers, in particular vegetable fibers, that is, fibers of vegetable origin, between 1% and 6% by weight. The fibers used in the embodiments described here are prebiotic fibers. They are the ideal substrate for the formation of endogenous SCFA by the intestinal microbiota.

In some embodiments, which can be combined with all the embodiments described here, the vegetable fibers can comprise soluble fibers and/or insoluble fibers.

The soluble vegetable fibers, preferable to be introduced in a liquid formulation, can be for example: inulin, FOS, GOS, resistant starch, pectin, guar gum, psyllium, acacia fiber or combinations thereof. All of these, while being or including carbohydrates, do not undergo transformation into glucose. Therefore, in the present description we will refer to vegetable fibers also including the fraction of carbohydrates, in particular non-metabolizable polysaccharides.

Such vegetable fibers can be advantageous for overcoming, or at least limiting, the disadvantages linked to the digestibility of known ketogenic food products.

In particular, vegetable fibers, although not directly metabolized by the body, perform a regulating effect on the intestinal flora, which would otherwise be impoverished and damaged by the low intake of carbohydrates. The prebiotic soluble fiber is in fact the ideal substrate for the formation of SCFAs by the intestinal microbiota, that is, for the formation of endogenous SCFA regardless of the possible addition or not of exogenous SCFA in the formulation described here.

In some embodiments, the food product can comprise further substances or ingredients, suitable to guarantee the desired consistency of the liquid food product and/or to provide further nutritional value and/or greater pleasantness to the taste.

These substances can comprise flavors, mineral salts, vitamin mixes, emulsifiers, sweeteners, thickeners.

In some embodiments described here, the emulsifiers and thickeners can be substances able to amalgamate and supply chemical stability to the liquid food product.

In some embodiments, the emulsifying substances can be surfactants able to promote and/or stabilize the emulsion phase of the food product.

In some embodiments, the liquid food product can have a lipid fraction comprised between 10% and 20% by weight with respect to the total weight of the product, and proteins can also be present between 5% and 10% by weight with respect to the total weight of the product.

In some embodiments, the liquid food product can exhibit a ketogenic ratio between lipids and sum of carbohydrates and proteins comprised between 1.5:1 and 2.5:1.

In such embodiments, the liquid food product alone can supply the necessary intake of nutrients provided by the ketogenic ratio.

In the liquid food product, the lipids can comprise unsaturated fatty acids, or their salts or their esters, in particular in the form of triglycerides of unsaturated fatty acids, between 1% and 3% by weight.

In some embodiments, the unsaturated fatty acids can comprise polyunsaturated fatty acids (PUFAs).

In some embodiments, the unsaturated fatty acids, in particular the polyunsaturated fatty acids (PUFAs), comprise linoleic acid (cis, cis-9, 12-octadecadienoic acid), or its salt or its ester, between 1% and 2% by weight. The linoleic acid can be supplied as linseed oil.

In some embodiments, the unsaturated fatty acids, in particular the polyunsaturated fatty acids (PUFAs), comprise alpha linolenic acid (octadeca-9Z, 12Z, 15Z-trienoic acid), or its salt or its ester, between 0.1% and 0.5%. The alpha linolenic acid can be supplied as safflower oil.

In some embodiments, the unsaturated fatty acids, in particular the polyunsaturated fatty acids, comprise docosahexaenoic acid (docosa-4Z, 7Z, 10Z, 13Z, 16Z, 19Z-hexaenoic acid, DHA), or its salt or its ester.

In the embodiments in which unsaturated fatty acids are present, the liquid food product can provide, as a source of polyunsaturated fatty acids (PUFAs), or their salts or their esters, one or more substances selected from: safflower oil, linseed oil, animal oil (for example fish oil) or vegetable oil (for example algae oil) rich in docosahexaenoic acid (DHA), or combinations thereof.

In some embodiments, the proteins comprise, or consist exclusively of, proteins of vegetable origin, in particular legume proteins, more in particular pea proteins.

In some embodiments, the proteins of vegetable origin, preferably pea proteins, are chosen for their hypoallergenic characteristics and for the same properties common to milk proteins ("Pea proteins oral supplementation promotes muscle thickness gains during resistance training: a double-blind, randomized, Placebo-controlled clinical trial vs. Whey protein", Babault et al., 2015). In this way, the food product described here can also be used by patients with food intolerances or allergies.

Furthermore, pea proteins are useful in reducing cholesterol levels in blood ("Hypocholesterolaemic effects of lupine protein and pea protein/fiber combinations in moderately hypercholesterolaemic individuals", Sirtori CR, Triolo M, Bosisio R, Bondioli A, Calabresi L, De Vergori V, Gomaraschi M, Mombelli G, Pazzucconi F, Zacherl C, Arnoldi A, Br. J. Nutr., 2012 Apr, 107(8): 1176-83, doi: 10.1017/S0007114511004120. Epub 2011 Oct 28).

### EXAMPLES

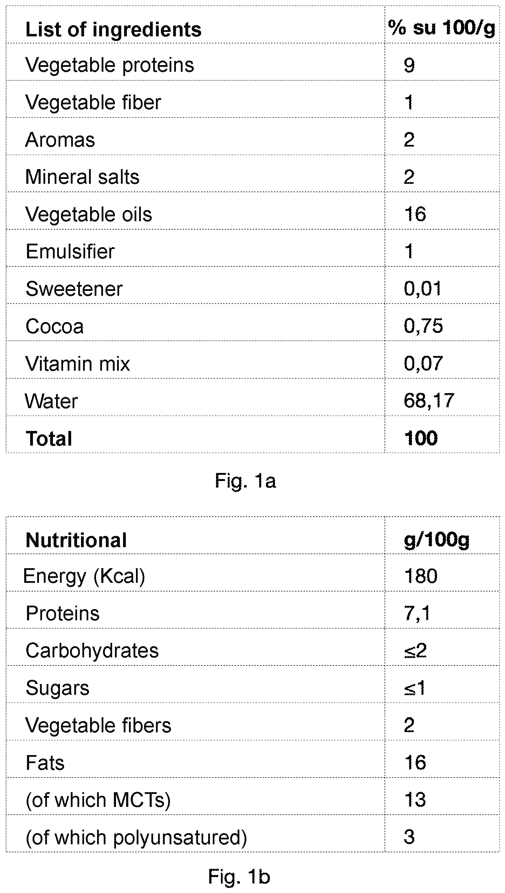

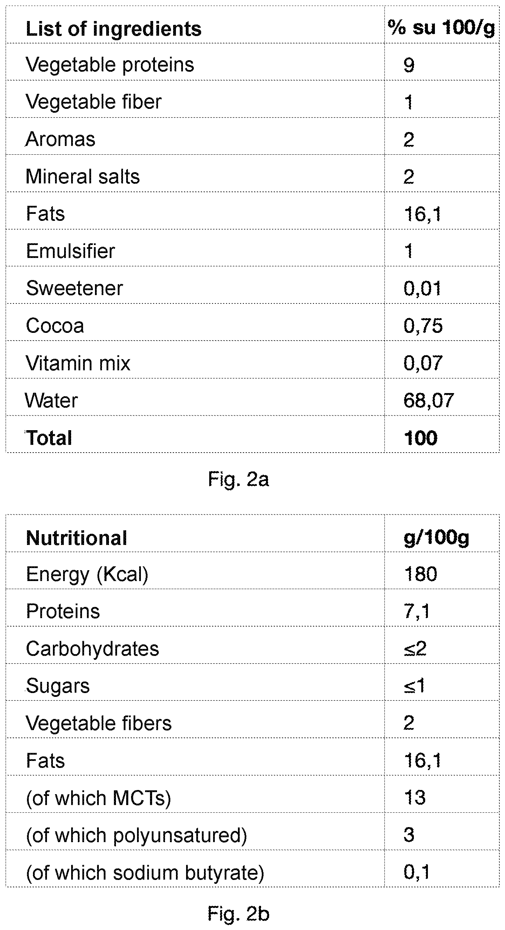

Some illustrations of example compositions and energetic and nutritional characteristics of liquid food product according to possible embodiments are present in figs. 1a and 1b (table 1a and 1b) and in figs. 2a and 2b (table 2a and 2b). In particular, tables 1a and 1b concern a product in which SCFAs are not present, while tables 2a and 2b concern a product in which SCFAs are present, in this case as sodium butyrate.

It is clear that modifications and/or additions of ingredients may be made to the liquid food product as described heretofore, without departing from the field and scope of the present invention as defined by the claims.

It is also clear that, although the present invention has been described with reference to some specific examples, a person of skill in the art shall certainly be able to achieve many other equivalent forms of liquid food product, having the characteristics as set forth in the claims and hence all coming within the field of protection defined thereby.

## Claims

1. Liquid food product suitable to manage the ketogenic diet and in particular to treat the symptoms of neurological diseases, said product comprising:
- lipids between 10% and 20% by weight with respect to the total weight of the product, of which short-chain fatty acids (SCFAs), or their salts or their esters, between 0% and 1% by weight with respect to the total weight of the product, and medium-chain triglycerides (MCTs), between 10% and 16% by weight with respect to the total weight of the product;
- carbohydrates less than 2% by weight with respect to the total weight of the product;
- fibers between 1% and 6% by weight with respect to the total weight of the product;
- water between 60% and 75% by weight with respect to the total weight of the product;
- proteins between 5% and 10% by weight with respect to the total weight of the product;
wherein said product has a ratio between lipids and the sum of carbohydrates and proteins between 1.5:1 and 2.5:1.

2. Product as in claim 1, **characterized in that** said short-chain fatty acids (SCFAs) are present between 0.1% and 1% by weight with respect to the total weight of the product.

3. Product as in claim 1 or 2, **characterized in that** said short-chain fatty acids (SCFAs) or their salts or their esters comprise butyric acid or its salt or ester.

4. Product as in claim 1, 2 or 3, **characterized in that** in the event said short-chain fatty acids (SCFAs) are supplied as esters, only one or more but not all, or all, of the substituent groups of ester are SCFAs.

5. Product as in claim 3 or in claim 4 when it depends on claim 3, **characterized in that** in the case of glycerol ester, only one or more but not all, or all, of the substituent groups which are bound to the glycerol in order to produce the ester are SCFAs.

6. Product as in claim 3 or as in claim 4 when it depends on claim 3 or as in claim 5, **characterized in that** the butyric acid salt is sodium butyrate.

7. Product as in any claim from 1 to 6, **characterized in that** said medium-chain triglycerides (MCTs) comprise medium-chain triglycerides (MCTs) deriving from decanoic acid, from octanoic acid and from lauric acid.

8. Product as in claim 7, **characterized in that** said product provides a ratio between MCTs deriving from octanoic acid and MCTs deriving from decanoic acid between 60:40 and 40:60.

9. Product as in any claim from 1 to 8, **characterized in that** said fibers are prebiotic fibers able to stimulate the bacterial flora to produce endogenous SCFA.

10. Product as in any claim from 1 to 9, **characterized in that** said lipids comprise unsaturated fatty acids, or their salts, or their esters, in particular polyunsaturated fatty acids (PUFAs), or their salts, or their esters, between 1% and 3% by weight with respect to the total weight of the product, wherein said polyunsaturated fatty acids (PUFAs) or their salts, or their esters, comprise one or more of:
- linoleic acid, or its salt or its ester, between 1% and 2% by weight with respect to the total weight of the product, and alpha linolenic acid, or its salt or its ester, between 0.1% and 0.5% by weight with respect the total weight of the product;
- docosahexaenoic acid (DHA), or its salt or its ester.

11. Product as in claim 10, **characterized in that** it comprises, as a source of said polyunsaturated fatty acids (PUFAs) or their salts, or their esters, one or more substances selected from the group comprising: safflower oil, linseed oil, animal or vegetable oil rich in docosahexaenoic acid (DHA), or combinations thereof.

12. Product as in any claim from 1 to 11, **characterized in that** said proteins comprise, or consist exclusively of, proteins of vegetable origin, in particular legume proteins, more particularly pea proteins.

## Patentansprüche

1. Flüssiges Lebensmittelprodukt, das geeignet ist, die ketogene Diät zu unterstützen und insbesondere Symptome neurologischer Erkrankungen zu behandeln, das Produkt umfasst:
- Lipide zwischen 10 und 20 Gewichts-% in Bezug auf das Gesamtgewicht des Produkts, davon kurzkettige Fettsäuren (SCFAs) oder deren Salze oder deren Ester, zwischen 0 und 1 Gewichts-% in Bezug auf das Gesamtgewicht des Produkts, und mittelkettige Triglyceride (MKT) zwischen 10 und 16 Gewichts-% in Bezug auf das Gesamtgewicht des Produkts;
- Kohlenhydrate weniger als 2 Gewichts-% in Bezug auf das Gesamtgewicht des Produkts;
- Fasern zwischen 1 und 6 Gewichts-% in Bezug auf das Gesamtgewicht des Produkts;
- Wasser zwischen 60 und 75 Gewichts-% in Bezug auf das Gesamtgewicht des Produkts;
- Proteine zwischen 5 und 10 Gewichts-% in Bezug auf das Gesamtgewicht des Produkts;
wobei das Produkt ein Verhältnis zwischen Lipiden und der Summe von Kohlenhydraten und Proteinen zwischen 1,5:1 und 2,5:1 aufweist.

2. Produkt nach Anspruch 1, **dadurch charakterisiert, dass** die kurzkettigen Fettsäuren (SCFAs) zwischen 0,1 und 1 Gewichts-% in Bezug auf das Gesamtgewicht des Produkts vorhanden sind.

3. Produkt nach Anspruch 1 oder 2, **dadurch charakterisiert, dass** die kurzkettigen Fettsäuren (SCFAs) oder deren Salze oder deren Ester Buttersäure oder dessen Salz oder Ester umfassen.

4. Produkt nach Anspruch 1, 2 oder 3, **dadurch charakterisiert, dass** in dem Fall der Bereitstellung der kurzkettigen Fettsäuren (SCFAs) als Ester nur eine oder mehrere, aber nicht alle, oder alle der Substituentengruppen des Esters SCFAs sind.

5. Produkt nach Anspruch 3 oder nach Anspruch 4, wenn es von Anspruch 3 abhängt, **dadurch charakterisiert, dass** in dem Fall von Glycerinester nur eine oder mehrere, aber nicht alle, oder alle Substituentengruppen, die an das Glycerin gebunden sind, um den Ester herzustellen, SCFAs sind.

6. Produkt nach Anspruch 3 oder nach Anspruch 4, wenn es von Anspruch 3 abhängt, oder nach Anspruch 5, **dadurch charakterisiert, dass** das Buttersäuresalz Natriumbutyrat ist.

7. Produkt nach einem der Ansprüche 1 bis 6, **dadurch charakterisiert, dass** die mittelkettigen Triglyceride (MKT) mittelkettige Triglyceride (MKT) umfassen, die sich von Decansäure, von Octansäure und von Laurinsäure ableiten.

8. Produkt nach Anspruch 7, **dadurch charakterisiert, dass** das Produkt ein Verhältnis zwischen MKT, die von Octansäure abgeleitet sind, und MKT, die von Decansäure abgeleitet sind, zwischen 60:40 und 40:60 bereitstellt.

9. Produkt nach einem der Ansprüche 1 bis 8, **dadurch charakterisiert, dass** die Fasern präbiotische Fasern sind, die fähig sind, die Bakterienflora zur Produktion endogener SCFA zu stimulieren.

10. Produkt nach einem der Ansprüche 1 bis 9, **dadurch charakterisiert, dass** die Lipide ungesättigte Fettsäuren oder deren Salze oder deren Ester umfassen, insbesondere mehrfach ungesättigte Fettsäuren (PUFAs) oder deren Salze oder deren Ester, zwischen 1 und 3 Gewichts-% bezogen auf das Gesamtgewicht des Produkts, wobei die mehrfach ungesättigten Fettsäuren (PUFAs) oder deren Salze oder deren Ester eine oder mehrere umfassen aus:
- Linolsäure oder dessen Salz oder dessen Ester, zwischen 1 und 2 Gewichts-% in Bezug auf das Gesamtgewicht des Produkts, und Alpha-Linolensäure oder dessen Salz oder dessen Ester, zwischen 0,1 und 0,5 Gewichts-% in Bezug auf das Gesamtgewicht des Produkts;
- Docosahexaensäure (DHA) oder dessen Salz oder dessen Ester.

11. Produkt nach Anspruch 10, **dadurch charakterisiert, dass** es als Quelle für die mehrfach ungesättigten Fettsäuren (PUFAs) oder deren Salze oder deren Ester eine oder mehrere Substanzen umfasst, ausgewählt aus der Gruppe umfassend: Distelöl, Leinsamenöl, Docosahexaensäure (DHA) reiches tierisches oder pflanzliches Öl oder Kombinationen hieraus.

12. Produkt nach einem der Ansprüche 1 bis 11, **dadurch charakterisiert, dass** die Proteine Proteine pflanzlichen Ursprungs umfassen oder ausschließlich daraus bestehen, insbesondere Hülsenfruchtproteine, genauer gesagt Erbsenproteine.

## Revendications

1. Produit alimentaire liquide adapté à la gestion du régime cétogène et en particulier au traitement des symptômes des maladies neurologiques, ledit produit comprenant :
- - des lipides entre 10 % et 20 % en poids par rapport au poids total du produit, dont des acides gras à chaîne courte (AGCC), ou leurs sels ou leurs esters, entre 0 % et 1 % en poids par rapport au poids total du produit, et des triglycérides à chaîne moyenne (TCM), entre 10 % et 16 % en poids par rapport au poids total du produit;
- - des hydrates de carbone inférieurs à 2 % en poids par rapport au poids total du produit ;
- des fibres entre 1% et 6% en poids par rapport au poids total du produit ;
- de l'eau entre 60% et 75% en poids par rapport au poids total du produit ;
- des protéines entre 5% et 10% en poids par rapport au poids total du produit ;
dans lequel ledit produit présente un rapport entre les lipides et la somme des hydrates de carbone et des protéines compris entre 1,5:1 et 2,5:1.

2. Produit selon la revendication 1, **caractérisé en ce que** lesdits acides gras à chaîne courte (AGCC) sont présents entre 0,1 % et 1 % en poids par rapport au poids total du produit.

3. Produit selon la revendication 1 ou 2, **caractérisé en ce que** lesdits acides gras à chaîne courte (AGCC) ou leurs sels ou leurs esters comprennent l'acide butyrique ou son sel ou ester.

4. Produit selon la revendication 1, 2 ou 3, **caractérisé en ce que** dans le cas où lesdits acides gras à chaîne courte (AGCC) ou leurs sels ou leurs esters sont fournis sous forme d'esters, seuls un ou plusieurs des groupes substituants de l'ester mais pas tous, ou tous, sont des AGCC.

5. Produit selon la revendication 3 ou la revendication 4 lorsqu'elle dépend de la revendication 3, **caractérisé en ce que** dans le cas de l'ester de glycérol, seulement un ou plusieurs groupes substituants mais pas tous, ou tous les groupes substituants qui sont liés au glycérol pour produire l'ester, sont des AGCC.

6. Produit selon la revendication 3 ou selon la revendication 4 lorsqu'elle dépend de la revendication 3 ou selon la revendication 5, **caractérisé en ce que** le sel d'acide butyrique est le butyrate de sodium.

7. Produit selon l'une quelconque des revendications 1 à 6, **caractérisé en ce que** lesdits triglycérides à chaîne moyenne (TCM) comprennent des triglycérides à chaîne moyenne (TCM) dérivés de l'acide décanoïque, de l'acide octanoïque et de l'acide laurique.

8. Produit selon la revendication 7, **caractérisé en ce que** ledit produit présente un rapport entre les TCM dérivant de l'acide octanoïque et les TCM dérivant de l'acide décanoïque entre 60:40 et 40:60.

9. Produit selon l'une des revendications 1 à 8, **caractérisé par le fait que** ces fibres sont des fibres prébiotiques capables de stimuler la flore bactérienne pour produire des AGCC endogènes.

10. Produit selon l'une quelconque des revendications 1 à 9, **caractérisé en ce que** lesdits lipides comprennent des acides gras insaturés, ou leurs sels, ou leurs esters, en particulier des acides gras polyinsaturés (AGPI), ou leurs sels, ou leurs esters, entre 1% et 3% en poids par rapport au poids total du produit, dans lequel lesdits acides gras polyinsaturés (AGPI) ou leurs sels, ou leurs esters, comprennent un ou plusieurs des éléments suivants :
- l'acide linoléique, ou son sel ou son ester, entre 1 % et 2 % en poids par rapport au poids total du produit, et l'acide alpha-linolénique, ou son sel ou son ester, entre 0,1 % et 0,5 % en poids par rapport au poids total du produit ;
- - l'acide docosahexaénoïque (DHA), ou son sel ou son ester.

11. Produit selon la revendication 10, **caractérisé en ce qu'**il comprend, comme source desdits acides gras polyinsaturés (AGPI) ou leurs sels, ou leurs esters, une ou plusieurs substances choisies dans le groupe comprenant : l'huile de carthame, l'huile de lin, l'huile animale ou végétale riche en acide docosahexaénoïque (DHA), ou des combinaisons de celles-ci.

12. Produit selon l'une quelconque des revendications 1 à 11, **caractérisé en ce que** lesdites protéines comprennent, ou sont constituées exclusivement, de protéines d'origine végétale, en particulier de protéines de légumineuses, plus particulièrement de protéines de pois.
